# FASCICULE DE BREVET EUROPEEN

(11) **EP 1 558 158 B1**
(45) Date de publication et mention de la délivrance du brevet: **16.03.2016**
(21) Numéro de dépôt: 03808758.1
(22) Date de dépôt: 13.10.2003
(51) Int. Cl.: A61B 17/70, A61B 17/80

(54) **DISPOSITIF DYNAMIQUE DE LIAISON INTERVERTEBRALE A DEBATTEMENT CONTROLE MULTIDIRECTIONNEL**
DYNAMISCHE ZWISCHENWIRBELVERBINDUNGSEINRICHTUNG MIT KONTROLLIERTEM MEHRRICHTUNGSAUSSCHLAG
DYNAMIC DEVICE FOR INTERVERTEBRAL LINKAGE WITH MULTIDIRECTIONAL CONTROLLED DISPLACEMENT

(30) Priorité: 14.10.2002 FR 0212726
(43) Date de publication de la demande: 03.08.2005
(73) Titulaire: SCIENT'X, 78284 Guyancourt (FR)
(72) Inventeur: CARLI, Olivier, CH-1207 Genève 6 (CH)
(74) Mandataire: Thibault, Jean-Marc
(86) Numéro de dépôt international: PCT/FR2003/003015
(87) Numéro de publication internationale: WO 2004/034916

(56) Documents cités:
- DE-C- 10 004 712
- FR-A- 2 814 936
- SU-A- 1 102 585
- US-A1- 2001 047 174
- US-B1- 6 267 764
- PATENT ABSTRACTS OF JAPAN vol. 1997, no. 08, 29 août 1997 (1997-08-29) & JP 09 108247 A (ROBAATO READ SHOKAI:KK), 28 avril 1997 (1997-04-28)

## Description

La présente invention concerne le domaine des dispositifs d'ostéosynthèse destinés notamment aux traitements de défauts ou d'états pathologiques de la colonne vertébrale ou des vertèbres.

L'objet de l'invention concerne plus particulièrement un dispositif de stabilisation intervertébrale permettant de maintenir en position relative convenable au moins deux vertèbres en vue de corriger chez un patient, par exemple, un tassement des vertèbres, une scoliose, une lordose, une cyphose ou une instabilité intervertébrale.

Dans le domaine d'application de l'ostéosynthèse du rachis, il existe de nombreux dispositifs d'ostéosynthèse comportant chacun un système de liaison relié de part et d'autre à des parties de fixation adaptées pour être fixées sur des vertèbres par l'intermédiaire d'éléments d'ancrage osseux.

Il est connu une première catégorie de dispositifs d'ostéosynthèse comportant un système de liaison rigide relié de part et d'autre aux parties de fixation sur les vertèbres. Un tel dispositif rigide de stabilisation conduit à un report des contraintes mécaniques sur les articulations intervertébrales adjacentes à celles stabilisées.

Pour remédier à ce problème, il est connu une deuxième catégorie de dispositifs d'ostéosynthèse comportant un système de liaison déformable élastiquement, relié de part et d'autre, aux parties de fixation sur les vertèbres. D'une manière générale, un tel dispositif de stabilisation intervertébrale dit dynamique comporte un système de type ressort ou amortisseur propre à résister élastiquement à un allongement et à une compression axiale. Un tel dispositif de stabilisation est capable d'amortir le mouvement en compression comme celui en traction autorisant un mouvement physiologique des segments vertébraux. Certains dispositifs connus sont adaptés également pour amortir les mouvements en flexion-extension dans le plan antéro-postérieur et les mouvements en flexion latérale.

FR 2814936 divulgue un dispositif selon le préambule de la revendication 1.

Il apparaît toutefois que les solutions antérieures connues ne sont pas de conception simple et présentent généralement un encombrement relativement important conduisant en particulier à une difficulté pour placer le dispositif dans une position non contrainte.

L'objet de l'invention vise donc à proposer un dispositif de liaison intervertébrale conçu pour amortir et contrôler les mouvements de compression-traction, de flexion-extension et d'inflexion latérale tout en étant de conception simple en présentant un encombrement réduit.

Pour atteindre un tel objectif, l'objet de l'invention concerne un dispositif d'ostéosynthèse pour colonne vertébrale comportant au moins un système de liaison déformable élastiquement relié de part et d'autre à au moins une première et une deuxième parties de fixation adaptées pour être fixées sur des vertèbres par l'intermédiaire d'éléments d'ancrage osseux.

Selon l'invention, le système de liaison déformable élastiquement comporte :
- un organe de liaison déformable comportant :
   - dans un plan dit sagittal une raideur déterminée pour exercer un effort de rappel pour des mouvements de flexion-extension des parties de fixation,
   - dans un plan dit frontal perpendiculaire au plan sagittal, une raideur déterminée pour exercer un effort de rappel pour des mouvements des parties de fixation d'inflexion latérale, la raideur de l'organe de liaison déformable dans le plan frontal étant inférieure à la raideur dans le plan sagittal,
   - selon un axe résultant qui est l'intersection entre le plan sagittal et le plan frontal, une raideur déterminée pour exercer un effort de rappel pour des mouvements de traction-compression des parties de fixation,
- et des moyens de limitation des mouvements de flexion-extension, de traction-compression et d'inflexion latérale entre les parties de fixation.

Diverses autres caractéristiques ressortent de la description faite ci-dessous en référence aux dessins annexés qui montrent, à titre d'exemples non limitatifs, des formes de réalisation de l'objet de l'invention.
La **fig. 1** est une vue en perspective d'un premier exemple de réalisation d'un dispositif d'ostéosynthèse conforme à l'invention.
La **fig. 2** est une vue en coupe élévation d'un dispositif d'ostéosynthèse tel qu'illustré à la **fig**. **1****.**
La **fig**. **3** est une vue partielle en perspective montrant le dispositif d'ostéosynthèse illustré à la **fig. 1** et **2** mais en position de traction.
La **fig**. **4** est une vue partielle en coupe d'un dispositif d'ostéosynthèse en position d'inflexion latérale.
La **fig**. **5** est une vue partielle montrant un dispositif d'ostéosynthèse en position de flexion.
La **fig**. **6** illustre un autre exemple de réalisation d'un dispositif d'ostéosynthèse conforme à l'invention.

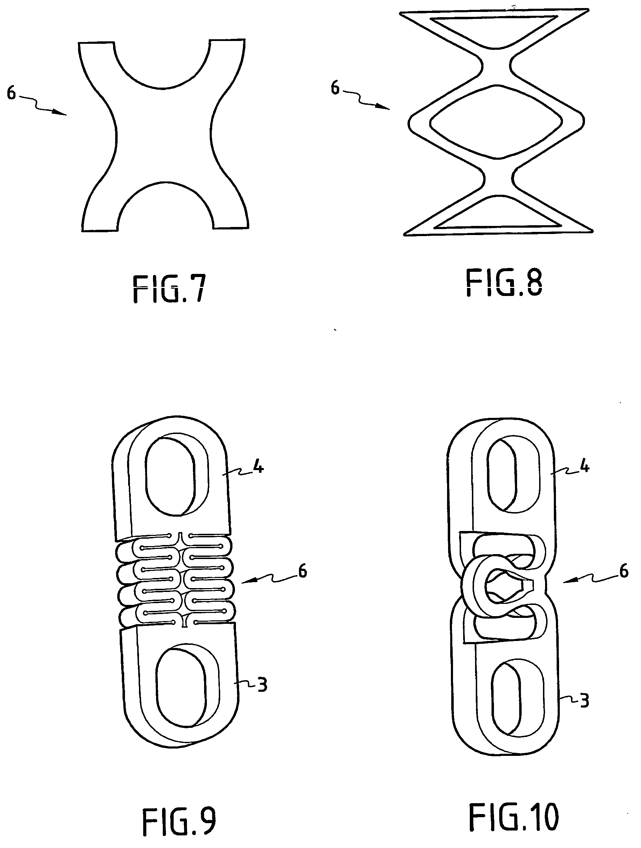

Les **fig. 7** à **10** illustrent diverses formes de réalisation d'un organe de liaison déformable faisant partie du dispositif d'ostéosynthèse conforme à l'invention.

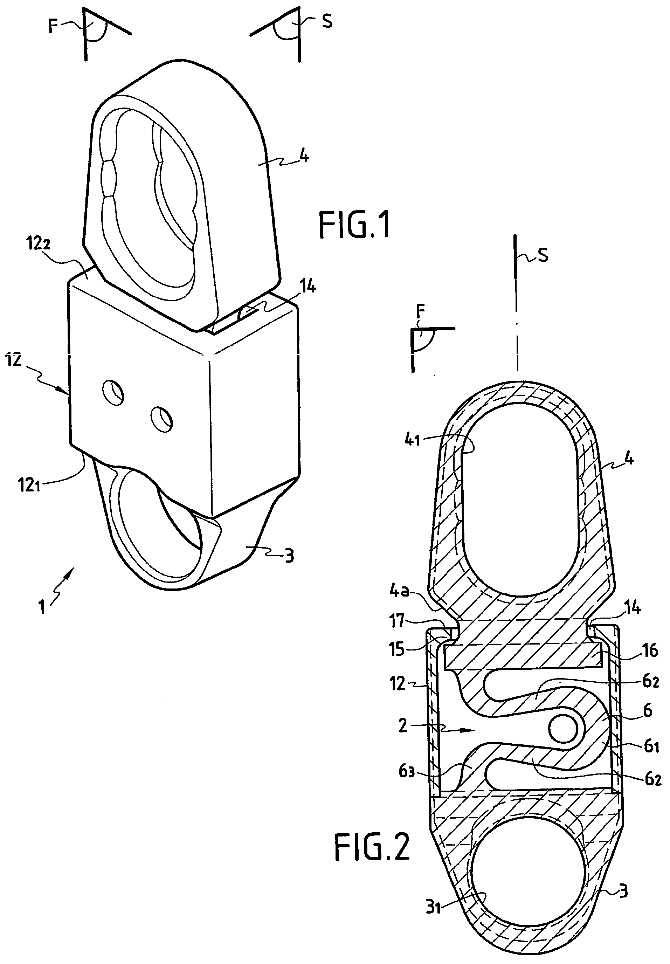

Tel que cela ressort plus précisément des **fig. 1** à **3**, l'objet de l'invention concerne un dispositif d'ostéosynthèse **1** pour la colonne vertébrale comportant au moins un système de liaison déformable élastiquement **2** relié de part et d'autre, à au moins une première partie de fixation **3** et à une deuxième partie **4** de fixation, adaptées pour être fixées sur des vertèbres par l'intermédiaire d'éléments d'ancrage osseux de tout type tels que des vis pédiculaires ou des crochets.

Conformément à l'invention, le système de liaison déformable élastiquement **2** comporte un organe de liaison déformable **6** comportant :
- dans un plan dit sagittal **S** en considération de l'anatomie, une raideur déterminée pour exercer un effort de rappel pour des mouvements de flexion-extension des parties de fixation **3, 4,**
- dans un plan dit frontal **F** perpendiculaire au plan sagittal **S**, une raideur déterminée pour exercer un effort de rappel pour des mouvements des parties de fixation **3, 4** d'inflexions latérales gauche ou droite,
- selon un axe résultant de l'intersection entre le plan sagittal **S** et le plan frontal **F,** une raideur déterminée pour exercer un effort de rappel pour des mouvements de traction-compression des parties de fixation **3, 4.**

Selon une caractéristique de l'invention, la raideur de l'organe de liaison déformable **6** dans le plan frontal **F** est inférieure à la raideur dans le plan sagittal **S.**

Dans la description qui précède, les mouvements des parties de fixation **3, 4** ont été décomposés de façon élémentaire. Bien entendu, après implantation du dispositif d'ostéosynthèse **1** selon l'invention, les mouvements du rachis imposés au dispositif obligent à une combinaison de ces mouvements dits élémentaires.

Dans l'exemple de réalisation illustré sur les dessins, l'organe de liaison déformable **6** est réalisé par l'intermédiaire d'un élément plat présentant un plan d'extension parallèle au plan frontal **F.** Cet élément plat **6** présente également une épaisseur qui s'étend dans un plan parallèle au plan sagittal **S.** Cet élément plat **6** qui constitue un ressort est conçu pour exercer un effort de rappel pour des mouvements de flexion-extension, de traction-compression et d'inflexion latérale pour les parties de fixation **3**, **4.**

Selon une variante préférée de réalisation, l'élément plat **6** présente dans le plan frontal **F** un profil courbe. D'une manière avantageuse, l'élément plat **6** présente dans le plan frontal **F,** un profil courbe en forme de lyre. Ainsi, tel que cela ressort plus précisément de la **fig. 2****,** l'élément plat **6** présente une âme arrondie **6₁** prolongée de part et d'autre par une branche **6₂** qui se termine par une partie d'extrémité incurvée **6₃** dirigée vers l'extérieur. Chaque partie incurvée **6₃** est reliée à une partie de fixation **3**, **4.** Dans l'exemple de réalisation illustré, l'élément plat **6** possède une section droite transversale en forme de quadrilataire.

Bien entendu, l'organe de liaison déformable **6** peut être réalisé de manière différente de la variante illustrée sur les dessins. Ainsi, par exemple, l'organe de liaison déformable **6** peut présenter une forme en **X (****fig. 7****)**, une partie en forme de losange raccordée de part et d'autre à une partie triangulaire (**fig. 8**), une succession simple ou comme illustré à la **fig. 9**, une double succession de plis et de contre-plis, ou une paire de lyres montées de façon inversée (**fig. 10**)**.**

Selon un aspect de l'invention, la mise en oeuvre d'un organe de liaison déformable présentant une forme différente et/ou une section différente offre l'avantage de disposer d'une gamme de raideur pour cet organe en relation de la physiologie du patient.

Selon une autre caractéristique avantageuse de l'invention, le système de liaison déformable élastiquement **2** comporte des moyens de limitation des mouvements de flexion-extension, traction-compression et d'inflexion latérale entre les parties de fixation **3**, **4**. De tels moyens permettent de contrôler l'amplitude des mouvements susceptibles d'être réalisés en traction-compression, en flexion-extension ou selon une inflexion latérale.

Dans un exemple préféré de réalisation, les moyens de limitation des mouvements entre les parties de fixation **3**, **4** sont constitués par des zones de butée présentées par un boîtier plat **12** coopérant avec des zones correspondantes de l'élément plat **6** monté à l'intérieur du boîtier **12**. Tel que cela ressort clairement des figures, le boîtier plat **12** se présente sous la forme d'un parallélépipède dont l'une des faces, par exemple **12₁,** est munie de la première partie de fixation **3.** La face **12₂** du boîtier, opposée à la face **12₁** équipée de la première partie de fixation **3**, possède un passage **14** pour la deuxième partie de fixation **4.** En d'autres termes, le passage **14** du boîtier présente une section supérieure à la section de la partie de fixation **4** pour permettre un débattement de cette partie de fixation **4.**

L'organe de liaison déformable **6** est ainsi monté à l'intérieur du boîtier **12** de part et d'autre duquel s'étendent les parties de fixation **3**, **4.** Bien entendu, le boîtier **12** est réalisé en au moins deux parties assemblées entre elles pour permettre le montage de l'organe de liaison déformable **6** à l'intérieur du boîtier **12.**

Tel que décrit précédemment, le boîtier **12** est adapté pour limiter les mouvements entre les parties de fixation **3**, **4.**

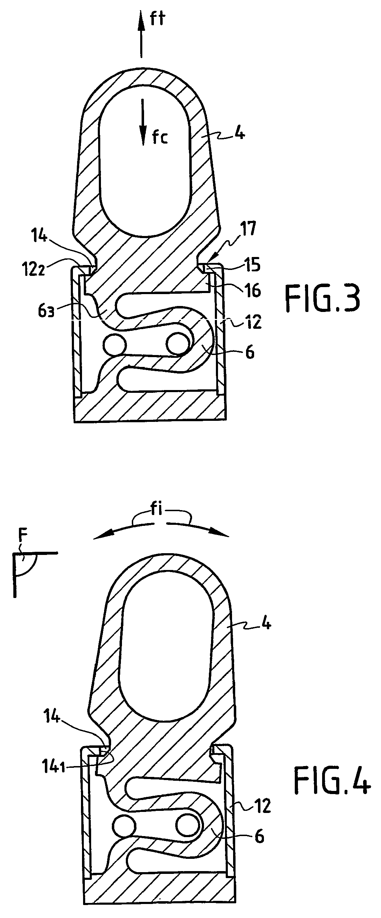

Tel que cela ressort plus précisément de la **fig. 3**, le boîtier **12** comporte un épaulement **15** sur lequel est destiné à venir en butée l'élément plat **6** lors d'un mouvement de traction exercé entre les parties de fixation **3, 4** et représenté par la flèche **ft.** Dans l'exemple illustré, l'épaulement **15** est délimité par la surface interne de la face **12₂** du boîtier bordant le passage **14**. Cet épaulement **15** est destiné à servir de butée pour une platine **16** sur laquelle est reliée une partie incurvée **6₃** de l'élément plat **6**. Bien entendu, la platine **16** possède une section droite transversale supérieure à celle du passage **14**.

De même, le boîtier **12** comporte une zone de butée **17** pour limiter un mouvement de compression exercé entre les parties de fixation **3, 4** et représenté par la flèche **fc** de sens opposé au sens de traction **ft.** Dans l'exemple illustré, la zone de butée **17** est formée par la surface externe de la face **12₂** du boîtier bordant le passage **14** et sur laquelle vient en contact une zone d'appui **4a** de la partie de fixation **4.**

Tel que cela ressort plus précisément de la **fig. 4**, le boîtier **12** est adapté pour limiter les mouvements d'inflexion latérale entre les parties de fixation **3, 4** s'établissant dans le plan frontal **F** et représentées par les flèches **fi.** Dans l'exemple illustré, l'organe de liaison déformable **6** est susceptible, lors de mouvement d'inflexion latérale, de venir en butée sur les bords transversaux **14₁** délimitant le passage **14.**

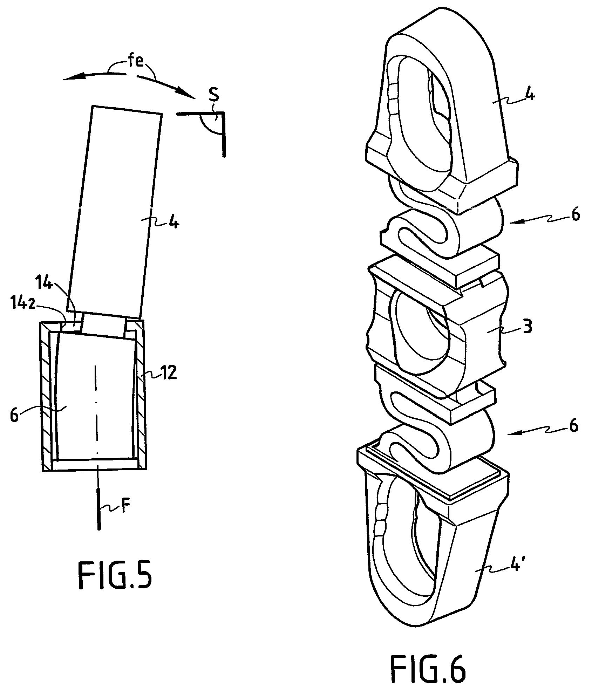

Comme cela ressort plus précisément de la **fig. 5**, le boîtier **12** est adapté pour limiter les mouvements de flexion-extension entre les parties de fixation **3, 4** s'établissant dans le plan sagittal **S** et représentés par les flèches **fe**. Dans l'exemple illustré, l'organe de liaison déformable **6** est susceptible, lors de mouvement de flexion-extension, de venir en butée sur les bords longitudinaux **14₂** délimitant le passage **14.**

Le boîtier **12**, par ses zones **15**, **17**, **14₁**, **14₂** constituant des butées, permet de limiter et contrôler les mouvements de traction-compression, d'inflexion latérale et de flexion-extension entre les parties de fixation **3**, **4.** Le dispositif d'ostéosynthèse selon l'invention permet d'amortir et de contrôler les débattements angulaires et axiaux autorisant des micro-mouvements.

Selon une caractéristique avantageuse de réalisation, le dispositif d'ostéosynthèse selon l'invention comporte des moyens permettant de régler sélectivement les mouvements entre les parties de fixation **3**, **4**. Ainsi, ces moyens de réglage peuvent sélectivement interdire soit le mouvement de flexion et/ou d'inflexion, soit d'inflexion gauche ou droite, soit de traction-compression. Ces moyens de réglage peuvent être réalisés, par exemple, par des pions placés à l'intérieur du boîtier **12** et jouant le rôle de butée sélective pour l'organe de liaison déformable **6** comme expliqué ci-dessus.

Selon une caractéristique préférée de réalisation, les parties de fixation **3**, **4**, présentent, sans contrainte extérieure, c'est-à-dire au repos, dans le plan sagittal **S** un décalage d'alignement propice à s'adapter aux angulations vertébrales.

Selon une variante préférée de réalisation, la première partie de fixation **3** est pourvue d'un trou de section droite transversale circulaire **3₁** de passage pour un élément d'ancrage. La réalisation d'un trou circulaire sur une partie de fixation **3** impose que deux dispositifs conformes à l'invention se trouvent positionnés symétriquement par rapport à un axe perpendiculaire au plan frontal, ce qui conduit à un fonctionnement physiologique des dispositifs selon l'invention. La deuxième partie de fixation **4** est pourvue d'un trou oblong **4₁** de passage pour un élément d'ancrage. La mise en oeuvre d'un trou oblong **4₁** autorise le montage à différentes positions d'un élément d'ancrage.

La **fig. 6** illustre un autre exemple de réalisation d'un dispositif d'ostéosynthèse **1** conforme à l'invention mettant en oeuvre un deuxième système de liaison déformable élastiquement **6** tel que décrit ci-dessus. Ce deuxième système de liaison déformable **6** est relié d'une part à la première partie de fixation **3** et d'autre part, à une troisième partie de fixation **4**' identique ou non à la deuxième partie de fixation **4.**

## Revendications

1. Dispositif d'ostéosynthèse (**1**) pour colonne vertébrale comportant au moins un système de liaison déformable élastiquement (**2**) relié de part et d'autre à au moins une première (**3**) et une deuxième (**4**) parties de fixation adaptées pour être fixées sur des vertèbres par l'intermédiaire d'éléments d'ancrage osseux,
**caractérisé en ce que** le système de liaison déformable élastiquement (**2**) comporte :
• un organe de liaison déformable (**6**) comportant :
- dans un plan dit sagittal (**S**) une raideur déterminée pour exercer un effort de rappel pour des mouvements de flexion-extension des parties de fixation,
- dans un plan dit frontal (**F**) perpendiculaire au plan sagittal, une raideur déterminée pour exercer un effort de rappel pour des mouvements des parties de fixation d'inflexion latérale, la raideur de l'organe de liaison déformable dans le plan frontal étant inférieure à la raideur dans le plan sagittal,
- selon un axe résultant qui est l'intersection entre le plan sagittal et le plan frontal, une raideur déterminée pour exercer un effort de rappel pour des mouvements de traction-compression des parties de fixation,
• et des moyens (**17**, **15**, **14₁**, **14₂)** de limitation des mouvements de flexion-extension, de traction-compression et d'inflexion latérale entre les parties de fixation (**3**, **4**).

2. Dispositif d'ostéosynthèse selon la revendication 1, **caractérisé en ce que** l'organe de liaison déformable (**6**) est réalisé par l'intermédiaire d'un élément plat présentant un plan d'extension dans le plan frontal (**F**) et une épaisseur s'étendant dans le plan sagittal (**S**), l'élément plat possédant au moins une première extrémité (**6₃**) reliée à la première partie de fixation (**3**) et une deuxième extrémité (**6₃**) reliée à la deuxième partie de fixation (**4**).

3. Dispositif d'ostéosynthèse selon la revendication 2, **caractérisé en ce que** l'élément plat (**6**) présente dans le plan frontal (**F**) un profil courbe de préférence en forme de lyre dont les extrémités (**6**₃) sont reliées aux parties de fixation.

4. Dispositif d'ostéosynthèse selon la revendication 2 ou 3, **caractérisé en ce que** l'élément plat (**6**) possède une section droite transversale en forme de quadrilatère.

5. Dispositif d'ostéosynthèse selon l'une des revendications 2 à 4, **caractérisé en ce que** l'élément plat (**6**) est placé à l'intérieur d'un boîtier plat (**12**) pourvu de la première partie de fixation (**3**) à laquelle est reliée une première extrémité de l'élément plat, le boîtier (**12**) comportant un passage (**14**) pour la deuxième partie de fixation s'étendant à l'extérieur du boîtier en étant reliée à la deuxième extrémité de l'élément plat (**6**).

6. Dispositif d'ostéosynthèse selon l'une des revendications 1 à 5, **caractérisé en ce que** les moyens (**17**, **15**, **14₁, 14₂)** de limitation des mouvements de flexion-extension, de traction-compression et d'inflexion latérale sont constitués par des zones de butée présentées par le boîtier (**12**) et coopérant avec des zones de l'élément plat (**6**).

7. Dispositif d'ostéosynthèse selon l'une des revendications 1 à 6, **caractérisé en ce que** les moyens de limitation des mouvements de flexion-extension sont constitués par les bords longitudinaux **(14₂)** de délimitation du passage (**14**) pour la deuxième partie de fixation (**4**).

8. Dispositif d'ostéosynthèse selon l'une des revendications 1 à 7, **caractérisé en ce que** les moyens de limitation des mouvements de traction-compression sont constitués d'une part, par un épaulement (**15**) aménagé sur le boîtier (**12**) et sur lequel est destinée à venir en butée une platine (**16**) réalisée sur la deuxième extrémité de l'élément plat (**6**) et d'autre part, par une zone de butée (**17**) aménagée sur le boîtier (**12**) et sur laquelle est destinée à venir en butée une zone d'appui (**4a**).

9. Dispositif d'ostéosynthèse selon l'une des revendications 1 à 8, **caractérisé en ce que** les moyens de limitation des mouvements d'inflexion latérale sont constitués par les bords transversaux **(14₁)** de délimitation du passage (**14**) pour la deuxième partie de fixation.

10. Dispositif d'ostéosynthèse selon l'une des revendications 1 à 9, **caractérisé en ce qu'**une première partie de fixation (**3**) est pourvue d'un trou circulaire de passage (**3₁**) pour un élément d'ancrage et qu'une deuxième partie de fixation (**4**) est pourvue d'un trou oblong de passage **(4₁)** pour un élément d'ancrage.

11. Dispositif d'ostéosynthèse selon l'une des revendications 1 à 10, **caractérisé en ce que** les parties de fixation (**3**, **4**) présentent dans le plan sagittal un décalage d'alignement.

12. Dispositif d'ostéosynthèse selon l'une des revendications 1 à 11, **caractérisé en ce qu'**il comporte un deuxième système de liaison déformable élastiquement (**6**) relié de part et d'autre à des parties de fixation (**3, 4'**).

13. Dispositif d'ostéosynthèse selon l'une des revendications 1 à 11, **caractérisé en ce qu'**il comporte des moyens permettant de régler sélectivement les mouvements entre les parties de fixation (**3, 4**).

## Patentansprüche

1. Wirbelsäulen-Osteosyntheseeinrichtung (1), umfassend mindestens ein elastisch verformbares Verbindungssystem (2), das auf beiden Seiten mit mindestens einem ersten (3) und einem zweiten (4) Fixierungsabschnitt verbunden ist, das angepasst ist, um mithilfe von Knochenverankerungselementen an Wirbeln befestigt zu werden,
**dadurch gekennzeichnet, dass** das elastisch verformbare Verbindungssystem (2) umfasst:
• ein verformbares Verbindungsorgan (6), umfassend:
- in einer so genannten Sagittalebene (S) eine bestimmte Steifheit, um eine Rückstellkraft für Flexions-Extensionsbewegungen der Fixierungsabschnitte auszuüben,
- in einer so genannten Frontalebene (F), senkrecht zur Sagittalebene, eine bestimmte Steifheit, um eine Rückstellkraft für Bewegungen der Fixierungsabschnitte zur seitlichen Inflexion auszuüben, wobei die Steifheit des in Frontalebene verformbaren Verbindungsorgans kleiner ist als die Steifheit in der Sagittalebene,
- gemäß einer resultierenden Achse, die der Schnittpunkt zwischen der Sagittalebene und der Frontalebene ist, eine bestimmte Steifheit, um eine Rückstellkraft für Zug-Kompressionsbewegungen der Fixierungsabschnitte auszuüben,
• und Mittel (17, 15, 14₁, 14₂) zur Begrenzung der Flexions-Extensions-, Zug-Kompressions- und seitlichen Inflexionsbewegungen zwischen den Fixierungsabschnitten (3, 4).

2. Osteosyntheseeinrichtung nach Anspruch 1, **dadurch gekennzeichnet, dass** das verformbare Verbindungsorgan (6) mithilfe eines flachen Elements ausgeführt wird, das eine Ausdehnungsebene in der Frontalebene (F) und eine Dicke, die sich in die Sagittalebene (S) erstreckt, aufweist, wobei das flache Element mindestens ein erstes Ende (6₃), das mit dem ersten Fixierungsabschnitt (3) verbunden ist, und ein zweites Ende (6₃), das mit dem zweiten Fixierungsabschnitt (4) verbunden ist, besitzt.

3. Osteosyntheseeinrichtung nach Anspruch 2, **dadurch gekennzeichnet, dass** das flache Element (6) in der Frontalebene (F) ein Kurvenprofil aufweist, bevorzugt in Form einer Lyra, deren Enden (6₃) mit den Fixierungsabschnitten verbunden sind.

4. Osteosyntheseeinrichtung nach Anspruch 2 oder 3, **dadurch gekennzeichnet, dass** das flache Element (6) einen Querschnitt in Form eines Vierecks besitzt.

5. Osteosyntheseeinrichtung nach einem der Ansprüche 2 bis 4, **dadurch gekennzeichnet, dass** das flache Element (6) im Innern eines flachen Gehäuses (12) angeordnet ist, das mit dem ersten Fixierungsabschnitt (3) versehen ist, mit dem ein erstes Ende des flachen Elements verbunden ist, wobei das Gehäuse (12) einen Durchgang (14) für den zweiten Fixierungsabschnitt umfasst, der sich außerhalb des Gehäuses erstreckt, indem er mit dem zweiten Ende des flachen Elements (6) verbunden ist.

6. Osteosyntheseeinrichtung nach einem der Ansprüche 1 bis 5, **dadurch gekennzeichnet, dass** die Mittel (17, 15, 14₁, 14₂) zur Begrenzung der Flexions-Extensions-, Zug-Kompressions- und seitlichen Inflexionsbewegungen aus Anschlagszonen bestehen, die von dem Gehäuse (12) dargestellt werden und die mit den Zonen des flachen Elements (6) zusammenwirken.

7. Osteosyntheseeinrichtung nach einem der Ansprüche 1 bis 6, **dadurch gekennzeichnet, dass** die Mittel zur Begrenzung der Flexions-Extensionsbewegungen aus den Längskanten (14₂) zur Begrenzung des Durchgangs (14) für den zweiten Fixierungsabschnitt (4) bestehen.

8. Osteosyntheseeinrichtung nach einem der Ansprüche 1 bis 7, **dadurch gekennzeichnet, dass** die Mittel zur Begrenzung der Zug-Kompressionsbewegungen einerseits aus einer Schulter (15), die auf dem Gehäuse (12) eingerichtet ist und an der vorgesehen ist, dass eine Platte (16) zum Anschlag kommt, die an dem zweiten Ende des flachen Elements (6) ausgeführt ist, und andererseits aus einer Anschlagszone (17), die auf dem Gehäuse (12) eingerichtet ist, und an der vorgesehen ist, dass eine Auflagezone (4a) in Anschlag kommt, bestehen.

9. Osteosyntheseeinrichtung nach einem der Ansprüche 1 bis 8, **dadurch gekennzeichnet, dass** die Mittel zur Begrenzung der seitlichen Inflexionsbewegungen aus den Querkanten (14₁) zur Begrenzung des Durchgangs (14) für den zweiten Fixierungsabschnitt bestehen.

10. Osteosyntheseeinrichtung nach einem der Ansprüche 1 bis 9, **dadurch gekennzeichnet, dass** ein erster Fixierungsabschnitt (3) mit einem kreisförmigen Durchgangsloch (3₁) für ein Verankerungselement versehen ist, und dass ein zweiter Fixierungsabschnitt (4) mit einem länglichen Durchgangsloch (4₁) für ein Verankerungselement versehen ist.

11. Osteosyntheseeinrichtung nach einem der Ansprüche 1 bis 10, **dadurch gekennzeichnet, dass** die Fixierungsabschnitte (3, 4) in der Sagittalebene einen Ausrichtungsversatz aufweisen.

12. Osteosyntheseeinrichtung nach einem der Ansprüche 1 bis 11, **dadurch gekennzeichnet, dass** sie ein zweites elastisch verformbares Verbindungssystem (6) umfasst, das auf beiden Seiten mit Fixierungsabschnitten (3, 4') verbunden ist.

13. Osteosyntheseeinrichtung nach einem der Ansprüche 1 bis 11, **dadurch gekennzeichnet, dass** sie Mittel umfasst, die es ermöglichen, die Bewegungen zwischen den Fixierungsabschnitten (3, 4) selektiv einzustellen.

## Claims

1. An osteosynthesis device (1) for the vertebral column, the device comprising at least one elastically deformable connection system (2) connected at opposite ends to at least first (3) and second (4) fixing portions suitable for being fixed to vertebrae by means of bone anchoring elements,
wherein the elastically deformable connection system (2) comprises:
- a deformable connection member (6) presenting:
- in a "sagittal" plane (5), determined stiffness for exerting a return force on flexion-extension movements between the fixing portions;
- in a "frontal" plane (F) perpendicular to the sagittal plane, determined stiffness for exerting a return force on lateral inflexion movements between the fixing portions, the stiffness of the deformable connection member in the frontal plane being less than its stiffness in the sagittal plane; and
- along an axis defined by the intersection between the sagittal and frontal planes, determined stiffness for exerting a return force on traction-compression movements between the fixing portions;
- and means (17, 15, 14₁, 14₂) for limiting flexion-extension, traction-compression, and lateral inflexion movements between the fixing portions (3, 4).

2. An osteosynthesis device according to claim 1, wherein the deformable connection member (6) is implemented by means of a flat element extending in the frontal plane (F) and presenting thickness in the sagittal plane (S), the flat element possessing at least a first end (6₃) connected to the first fixing portion (3) and a second end (6₃) connected to the second fixing portion (4).

3. An osteosynthesis device according to claim 2, wherein the flat element (6) presents a curved profile in the frontal plane (F), the curved profile preferably being lyre-shaped with its ends (6₃) being connected to the fixing portions.

4. An osteosynthesis device according to claim 2 or 3, wherein the flat element (6) presents a right cross-section that is quadrangular in shape.

5. An osteosynthesis device according to claim 2, wherein the flat element (6) is placed inside a flat box (12) provided with the first fixing portion (3) which is connected to a first end of the flat element, the box (12) having a passage (14) for the second fixing portion which projects outside the box and which is connected to the second end of the flat element (6).

6. An osteosynthesis device according to one of claims 1 to 5, wherein the means (17, 15, 14₁, 14₂) for limiting flexion-extension, traction-compression, and lateral inflexion movements are constituted by abutment zones presented by the box (12) and co-operating with zones of the flat element (6).

7. An osteosynthesis device according to one of claims 1 to 6, wherein the means for limiting flexion-extension movements are constituted by the longitudinal edges (14₂) defining the passage (14) for the second fixing portion (4).

8. An osteosynthesis device according to one of claims 1 to 7, wherein the means for limiting traction-compression movements are constituted firstly by a shoulder provided on the box and against which a platen (16) made on the second end of the flat element (16) is designed to come into abutment, and secondly by an abutment zone (17) provided on the box (12) and against which a bearing zone (4a) is designed to come into abutment.

9. An osteosynthesis device according to one of claims 1 to 8, wherein the means for limiting lateral inflexion movements are constituted by transverse edges (14₁) defining the passage (14) for the second fixing portion.

10. An osteosynthesis device according to one of claims 1 to 9, wherein a first fixing portion (3) is provided with a circular through hole (3₁) for passing an anchoring element, and wherein a second fixing portion (4) is provided with an oblong through hole (4₁) for passing an anchoring element.

11. An osteosynthesis device according tone of claims 1 to 10, wherein the fixing portions (3,4) present an alignment offset in the sagittal plane.

12. An osteosynthesis device according to one of claims 1 to 11, further comprising a second elastically deformable connection system (6) connected at opposite ends to fixing portions (3,4').

13. An osteosynthesis device according to one of claims 1 to 11, including means enabling movements between the fixing portions (3,4) to be adjusted selectively.
